(19) Europäisches Patentamt
European Patent Office
Office européen des brevets

(11) Publication number: 0 119 332
B1

(12) **EUROPEAN PATENT SPECIFICATION**

(45) Date of publication of patent specification: **25.05.88**

(51) Int. Cl.⁴: **C 12 N 9/04**, C 12 Q 1/26

(21) Application number: **83301376.6**

(22) Date of filing: **11.03.83**

(54) **Novel pyranose oxidase processes for the production and use thereof and test compositions for use therein.**

(43) Date of publication of application:
**26.09.84 Bulletin 84/39**

(45) Publication of the grant of the patent:
**25.05.88 Bulletin 88/21**

(84) Designated Contracting States:
**DE FR GB IT**

(56) References cited:
**EP-A-0 054 358**
**WO-A-81/03666**

**CHEMICAL ABSTRACTS, vol. 99, no. 1, 4th July 1983, page 392, no. 4091x, Columbus, Ohio, USA**

**CHEMICAL ABSTRACTS, vol. 93, no. 7, 18th Agust 1980, page 514, no. 66196v, Columbus, Ohio, USA J. VOLC et al.: "Glucose-2-oxidase activity and accumulation of D-arabino-2-hexosulose in cultures of the basidiomycete Oudemansiella mucida" & FOLIA MICROBIOL. (PRAGUE) 1978, 23(4), 292-298**

(73) Proprietor: **KYOWA HAKKO KOGYO CO., LTD.**
**Ohtemachi Bldg., 6-1 Ohtemachi I-chome**
**Chiyoda-ku Tokyo 100 (JP)**

(72) Inventor: **Nakanishi, Toru**
**145-42 Tomuro**
**Atsugi-shi Kanagawa-ken (JP)**
Inventor: **Machida, Yozo**
**500-50, Arai Hodogaya-ku**
**Yokohama-shi Kanagawa-ken (JP)**

(74) Representative: **Thomas, Roger Tamlyn et al**
**D. Young & Co. 10 Staple Inn**
**London WC1V 7RD (GB)**

(56) References cited:
**BIOCHIMICA AND BIOPHYSICA ACTA, vol. 167, 1968, pages 501-510 F.W. JANSSEN et al.: "Carbohydrate oxidase, a novel enzyme from Polyporus Obtusus. II. Specificity and characterization of reaction products"**

**EP 0 119 332 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to the preparation and use of pyranose oxidases and, in particular, to the production of a novel pyranose oxidase exhibiting a strong specific activity on glucose alone.

In the prior art a pyranose oxidase (EC 1.1.3.10) is known which catalyzes the oxidation of glucose, xylose and sorbose into glycosone, xylosone and 5-ketofructose, respectively (methods in Enzymol, vol. XLI, p. 170, (1975)). In oxidizing these substances, oxygen is consumed and hydrogen peroxide is formed and the enzyme is, therefore, useful for the total quantitative determination of pyranoses such as glucose, xylose and sorbose but without distinction between them. This known enzyme is produced by culturing a microorganism belonging to the genus *Polyporus* (Methods in Enzymol, Vol. XLI p. 170 (1975)).

In contrast, the present invention provides a novel pyranose oxidase which shows strong specific activity which is substantially restricted to activity on glucose alone.

According to the present invention, this novel pyranose oxidase is obtained by culturing a microorganism belonging to the genus *Coriolus, Daedaleopsis, Gloeophyllum* or *Pleurotus* and which is capable of producing the novel pyranose oxidase, in a nutrient medium until enzymatic activity is detected in the culture liquor and thereafter recovering the pyranose oxidase therefrom.

The novel enzyme of this invention is useful for the quantitative determination of pyranose oxidase substrates, especially D-glucose, by oxidizing the substrate in the presence of oxygen and measuring the amount of a product of the reaction, usually the hydrogen peroxide, or the amount of oxygen consumed by said reaction.

In the following description of the properties of the novel enzyme of this invention, the following method is used for the determination of the enzymatic activity.

*Assay Method*

A 3.0 ml sample is first prepared comprising 1.0 ml of 0.1 M trishydrochloride buffer (pH 7.0), 0.2 ml of a colouring reagent prepared in advance as hereinafter described, 0.1 ml of 1 M glucose solution, 0.1 ml of a solution of the pyranose oxidase at a known concentration, 0.1 ml of 0.1 mM flavinadenine dinucleotide and 1.5 ml of distilled water. Said colouring reagent is prepared by adding 0.1 M tris-hydrochloride buffer (pH 7.0) containing 10 mmol/l 4-aminoantipyrine (hereinafter referred to as 4AA) and 10 mmol/l phenol, to an amount of a peroxidase which is sufficient to provide a peroxidase concentration in said buffer of 2000 U per 100 ml. The pyranose oxidase containing sample is incubated at 37°C and the increasing rate of the absorbancy at 500 nm is automatically measured on a spectrophotometer. If the molecular extinction coefficient of a quinoneimine pigment formed under these conditions is defined as $5.3 \times 10^3$, and one unit of enzymatic activity is defined as the amount of the enzyme that produces one µmole of hydrogen peroxide per minute at a temperature of 37°C and a pH of 7.0, then the enzymatic activity (A) of the pyranose oxidase in the sample can be defined by the following formula:—

$$A = \frac{\Delta A500}{5.3} \times \frac{3.0}{0.1} \times \text{the dilution rate of the enzyme}$$

where A500 is the rate of increase of the absorbancy of the sample per minute at 500 nm.

The properties of the novel enzyme of this invention are also described hereinafter with reference to the accompanying drawings, in which:—

Figure 1 shows the relationship between the relative activity of the enzyme and the stable pH range.

Figure 2 shows the relationship between the relative activity of the enzyme and the optimum pH.

Figure 3 shows the relationship between the relative activity of the enzyme and the optimum temperature.

Figure 4 shows the relationship between the relative activity of the enzyme and the heat stability.

Figure 5 shows the relationship between the absorbancy and the glucose concentration.

In further detail, the properties of the novel enzyme of this invention are:—

(1) *Stable pH range*

Samples of the enzyme were dissolved in three different buffers, to make up enzyme solutions having an activity of 100 mU/0.7 ml. viz a citrate buffer, a Tris buffer and a borate buffer. In this test buffer solutions were used having the pH values indicated in Figure 1. Each enzyme solution was incubated at 50°C for 30 minutes, cooled and neutralized prior to measurement of the residual enzymatic activity by the above described assay method.

The present enzyme is stable at a pH of 5.0—7.4 as shown in Figure 1.

(2) *Optimum pH*

Samples of the enzyme were dissolved in Tris buffer solutions have the pH indicated in Figure 2 to make up enzyme solutions having an activity of 250 mU/3.7 ml. Each of the enzyme solutions was put into a BIOXYGRAPH cell (trade name for a cell for measuring oxygen concentration made by Kyusui Kagaku Laboratory, Japan), and 50 mL of 1 M glucose were added. Measurements were made to determine the amount of absorbed oxygen. The optimum pH for this enzyme was shown to be around 6.2.

(3) *Optimum temperature*

The same enzymatic reactions as described in the assay method were repeated for 5 minutes at the temperatures indicated in Figure 3, except that pyranose oxidase concentrations of 100 mU were used. The optimum temperature for the present enzyme was determined to be 50°C.

(4) *Heat stability*

The enzyme was dissolved in 0.6 ml of 0.2 M Tris buffer solution (pH 6.0) to make up an enzyme solution having an activity of 100 mU/0.7 ml. The enzyme solution was incubated for 30 minutes at the temperatures indicated in Figure 4 and measurements made to measure the residual enzymatic activity. As shown, the enzyme keeps 90% of its activity with treatment at 60°C for 30 minutes.

(5) *Substrate specificity*

The same reactions as described in the assay method for enzymatic activity were repeated except that an enzyme concentration of 2.5 U/ml was used, and, as the substrate, 0.1 ml samples of various carbohydrate liquors indicated in Table 1 were used, each such liquor having a concentration of 100 mM. The results are expressed as percentages of the glucose reaction and as will be seen the specificity of this enzyme with glucose is very high and substantially exclusive thereto with only minor levels of activity on mannose, galactose, sorbose and xylose. The specificity with respect to the latter two, in particular being a great deal lower than that of the known pyranose oxidase derived from microorganisms of the genus *Polyporus* (Table 1 of B.B.A. *167*, 501—510 (1968)).

TABLE 1

| Substrate | Relative Activity (%) | Substrate | Relative Activity (%) |
|---|---|---|---|
| glucose | 100 | maltose | 0 |
| mannose | 0.9 | trehalose | 0 |
| galactose | 0.9 | lactose | 0 |
| sorbose | 3.4 | mannitol | 0 |
| fructose | 0 | glucosamine | 0 |
| xylose | 2.6 | gluconic acid | 0 |

(6) *Electron acceptor*

In this phase, 1.4 ml of 50 mM tris-hydrochloride buffer (pH 7.0), 0.1 ml of 250 mU enzyme solution and 0.5 ml of electron acceptor solutions having various concentrations as indicated in Table 2 were put into a cell with Thunberg tube. After saturating with dissolved oxygen, the reaction solution was mixed with 0.5 ml of a 0.1 M glucose solution. The change in absorbancy per minute from 30 minutes to 90 minutes after mixing was determined and the activities of the electron acceptors are expressed as relative activity. As will be seen the optimum electron acceptor for this enzyme is oxygen.

TABLE 2

| Electron Acceptor | Concentration (mM) | Relative Activity (%) |
|---|---|---|
| Oxygen | Dissolved | 100 |
| 2,6-Dichlorophenolindolphenol | 0.04 | 2.5 |
| Nitroprusside-tetrazolium | 0.1 | 0 |
| Cytochrome C | 0.1 | 0.33 |
| Nicotinamide-adenine dinucleotide | 0.1 | 0 |
| Nicotinamide-adenine dinucleotide phosphate | 0.1 | 0 |
| Ferricyanide compound | 0.5 | 0 |

(7) *Enzyme inhibition*

Various inhibitors were studied in the same manner as that adopted in determination of the optimum pH. A reaction solution comprising 3.4 ml of 50 mM tris-hydrochloride buffer (pH 7.0), 0.1 ml of enzyme solution containing 280 mU of pyranose oxidase and 0.2 ml of 18.5 mM inhibitor solution were put into a cell for measuring oxygen uptake and 50 µl of 1M glucose were added thereto and the sample incubated at 37°C. The results are given in Table 3. As shown the enzymatic action is substantially inhibitied by $Cu^{2+}$, $Ag^{+}$, $Ni^{2+}$ and p-chloromercuribenzoate, and to a lesser extent by $Co^{2+}$, 8-hydroxyquinoline and hydrogen peroxide, and not at all by $Mg^{2+}$.

TABLE 3

| Inhibitor | Percent Inhibition (%) |
|---|---|
| magnesium sulfate | 0 |
| cobalt sulfate | 13.3 |
| copper sulfate | 35.7 |
| silver nitrate | 53.3 |
| nickel chloride | 28.6 |
| 8-hydroxyquinoline | 13.3 |
| hydrogen peroxide | 20.0 |
| p-chloromercuribenzoate | 50.0 |

(8) *Isoelectric point*

The isoelectric point of the enzyme is 4.2 as determined by the isoelectric focussing method using carrier ampholyte at a pH of 3.5—10.0.

(9) *Molecular weight*

By the gel-filtration method on Sephadex G—200 (Registered Trade Mark) and using, as the standard protein, egg albumin, bovine blood serum, alcohol dehydrogenase, γ-globulin and xanthin oxidase derived from milk, the molecular weight of the enzyme is calculated to be about 220,000.

(10) *Km value*

According to the Lineweaver-Burk plot method using 2.5 U pyranose oxidase the Km value for glucose is determined to be 0.83 mM, which is about one-twelfth to one-twenty fourth of that of glucose oxidase (10—20 mM).

The pyranose oxidase of the present invention is produced as follows.

Any microorganism may be used in the present invention so long as it belongs to the genus *Coriolus, Daedaleopsis, Pleurotus* or *Gloephyllum* and is capable of producing pyranose oxidase. Examples of the preferred strains are *Coriolus versicolor* ATCC 20155, *Daedaleopsis styracina* ATCC 20188, *Gloephyllum sepiarium* Z—41, NRRL 12506 and *Pleurotus ostreatus* Z—64, NRRL 12507.

These strains have been deposited with American Type Culture Collection in U.S.A. or Northern Regional Research Laboratory in U.S.A. under the above deposit numbers and are publicly available from those collections.

The mycological properties of the strain are described in the following literature.

| | |
|---|---|
| *Coriolus versicolor*<br>*Daedaleopsis styracina*<br>*Gloeophyllum sepiarium* | Nihon Kinrui Shi (Japanese Microorganisms) Vol. 4, No. 2, (1955) by Seiya Ito |
| *Pleurotus ostreatus*: | Genshoku Nihon Kinrui Zukan (Primary Color Japanese Microorganism Pictorial Book) (1957) by Imazeki and Hongo |

Either a synthetic or natural medium may be used as long as it properly contains a carbon source, a nitrogen source, minerals and other nutrients.

As the carbon source, various carbohydrates such as glucose, fructose, sucrose, maltose, mannose, starch, starch hydrolyzate liquor, molasses, etc., various sugar alcohols such as glycerol, sorbitol, mannitol, etc., various organic acids such as acetic acid, lactic acid, pyruvic acid, fumalic acid, citric acid, etc., various alcohols such as methanol, ethanol, etc., various glycols such as ethylene glycol, propylene glycol, etc., various amino acids, and hydrocarbons such as n-hexadecane, etc., may be used.

As the nitrogen source, there may be employed ammonia, various inorganic and organic ammonium salts such as ammonium chloride, ammonium carbonate, ammonium phosphate, ammonium nitrate, ammonium acetate, etc., urea, amino acids and other nitrogen-containing compounds, as well as nitrogenous organic materials such as peptone, NZ-amine, meat extract, corn steep liquor, casein hydrolyzate, chrysalis hydrolyzate, fish meal, its digest product, defatted soybean, its digest product, etc.

As the minerals, appropriate are potassium primary phosphate, potassium secondary phosphate, potassium chloride, magnesium sulfate, manganese sulfate, ferrous sulfate, sodium chloride, calcium carbonate, etc.

Culturing is continued until the enzymatic activity is detected in the culture liquor, mostly within the microbial cells, generally for 2—4 days. Recovering of pyranose oxidase from the culture liquor is carried out as follows:

After completion of culturing, the microbial cells are collected from the culture liquor by filtration and disrupted by suitable means to obtain a cell extract. The extract is subjected to centrifugation to obtain a supernatant. Then, the supernatant is subjected to conventional purification methods such as salting-out, precipitation with organic solvent, dialysis, ion exchange cellulose column chromatography, gel-filtration method and freeze-drying. Thus, purified pyranose oxidase can be recovered.

Besides the novel enzyme of this invention, and a process for its preparation, the present invention also provides a method and test composition or kit for the determination of the substrates for pyranose oxidase in a sample, the test composition or kit comprising (A) a component for oxidizing the substrate and comprising the novel pyranose oxidase and (B) an apparatus or component for determining the reaction product or the amount of oxygen consumed in the enzymatic reaction component B, in particular, comprising a peroxidase and a chromogen reactive with hydrogen peroxide to produce a coloured product which can be determined colorimetrically.

The determination method of the present invention is based on the following principle. When the substrate for pyranose oxidase is oxidized by the action of pyranose oxidase, the amount of the reaction product or the amount of consumed oxygen is directly proportional to the amount of the substrate in a sample. The amount of oxygen consumed, or the amount of product produced can thus be used as a measure of the substrate, and particularly, in the case of the present invention, glucose since the present enzyme exhibits a strong specific activity only on glucose.

In the case of glucose, the enzyme reaction is schematically below.

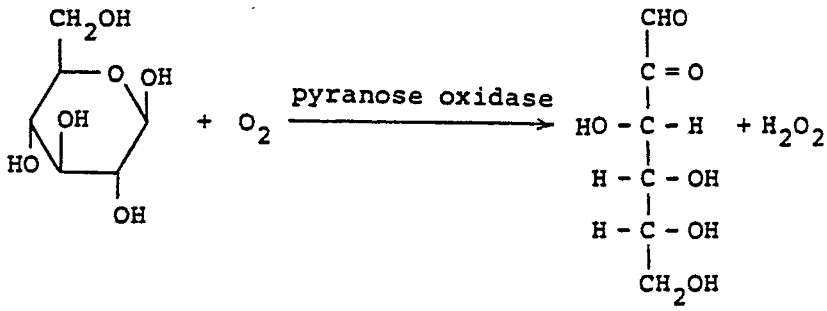


β-D-glucose glucosone

As well as the direction determination of glucose the present invention can also be applied to the determination of other compounds which quantitatively give rise to glucose in the reaction system. For example, the present invention can be used in the determination of glucose derivatives such as maltose and methyl glucoside; enzymes such as glucosidase, maltose phosphoryrase, malate dehydrogenase, glucose-6-phosphatase, amylase, glucos-1-phosphatase and trehalase, and reduced nicotinamide-adeninedinucleotide (phosphate) (NAD (P) $H_2$) and ADP.

The enzymatic reaction for the determination of these compounds or enzymes are shown below.

(1) For amylase, maltose, maltosephosphorylase (MPR)

$$G_5 \xrightarrow{\alpha\text{-Amylase}} G_3 + \text{maltose}$$

$$\text{maltose} + H_3PO_4 \xrightarrow{\text{MPR}} \beta\text{-D-glucose-1-phosphate} + \text{glucose}$$

↓

Detection

(2) For NAD(P)$H_2$

$$NAD(P)H_2 + \text{D-glucose-}\delta\text{-lactone}$$

$$\xrightarrow{\text{GDH}} NAD(P) + \beta\text{-D-glucose}$$

↓

Detection

GDH: glucose dehydrogenase

(3) For malate dehydrogenase (MDH)

$$\text{2-Malic acid} + NAD(P) \xrightarrow{\text{MDH}} \text{Pyruvic acid} + NAD(P)H_2$$

↓

Detection

(4) For ADP

$$ADP + \text{D-glucose-6-phosphate} \xrightarrow{\text{glucokinase}} ATP$$

$$+ \text{D-glucose}$$

↓

Detection

(5) For glucose-6-phosphatase (G6P)

$$\text{D-glucose-6-phosphate} + H_2O \xrightarrow{\text{G6P}} \text{D-glucose} + H_3PO_4$$

(6) For glucose-1-phosphatase (G1P)

$$\text{D-glucose-1-phosphate} + H_2O \xrightarrow{G1P} \text{D-glucose} + H_3PO_4$$

(7) For trehalase

$$\text{Trehalose} + H_2O \xrightarrow{\text{trehalase}} \text{2D-glucose}$$

According to the present invention, the determination of the substrate may be performed by conducting the individual reaction steps individually or together and may preferably be performed by subjecting the sample to enzymatic reaction and a colourforming reaction with a reagent comprising pyranose oxidase and a detecting system for at least one of the products formed by the enzymatic reaction. An example of a preferred detecting system is a hydrogen peroxide detecting system which comprises peroxidase and a chromogen.

Several methods for the determination of hydrogen peroxide are known. Representative methods include:

(1) reacting hydrogen peroxide with a chromogen in the presence of peroxidase to form a pigment and determining the pigment colorimetrically;

(2) reacting hydrogen peroxide with alcohol in the presence of catalase to form an aldehyde, reacting the aldehyde with acetyl acetone and ammonia to form a pigment and determining the pigment colorimetrically; and

(3) reacting hydrogen peroxide with alcohol in the presence of catalase to form an aldehyde, reacting the aldehyde with the reduced form of nicotinamide adenine dinucleotide (hereinafter referred to as NADH) to form nicotinamide adenine dinucloeotide (hereinafter referred to as NAD) and determining NAD colorimetrically.

Among these methods, the method using peroxidase is very simple and the determination of hydrogen peroxide is performed by measuring the absorbancy of the reaction solution based on the color development of formed pigment with a spectrophotometer.

In determining a substrate for pyranose oxidase, a sample containing the substrate and pyranose oxidase are added to an appropriate buffer at a pH of 7.0—8.0 and the mixture is subjected to reaction. Pyranose oxidase may be microencapsulated or immobilized by a carrier, if necessary.

As a buffer solution, borate buffer, phosphate buffer, tris-hydrochloride buffer, tris-maleate-hydrooxide buffer, citrate-disodium phosphate buffer, succinate-borate buffer, phosphate-borate buffer and the like, are used in a concentration of 0.005—0.5 mol/l. The enzyme is generally used in a concentration of 0.1—10 U/ml.

Examples of suitable chromogens are 4-aminoantipyrine (4AA)-phenol, 4AA-dimethylaniline (DMA), 4AA-diethylaniline (DEA), 4AA-N-ethyl-N-meta-methylphenyl-N'-acetylethylenediamine (EMAE), 3-methyl-2-benzothiazoline-hydrazone-HCl (MBTH)-phenol, MBTH-DEA, 4AA-dibutylaniline (DBA), MBTH-DBA, 4AA-3,5-dimethoxy-N-(β-hydroxy-γ-sulphopropyl)-aniline (DHSA), etc.

Reaction is carried out in the presence of peroxidase and a chromogen in a concentration of 0.5—10 U/ml and 1—20 μmol/ml, respectively, at a temperature of 30—50°C, and continued for 5—20 minutes. After completion of the enzymatic reaction, the absorbancy of the reaction solution is measured at a visible ray region, generally at 400—600 nm. The relationship between the amount of a substrate and the absorbancy is given in advance in the form of calibration curve prepared using a standard solution of the substrate. Then, the substrate contained in a sample is determined from the absorbancy of the sample.

The present invention also relates to a test composition for determination and a kit utilizing thereof. The test composition comprises the novel pyranose oxidase, peroxidase and a chromogen and the kit is made of the test composition and a buffer solution. The test composition may contain an enzyme or decomposition reagent necessary for forming glucose. For example, a test composition for determining α-amylase contains starch and maltose phosphorylase.

Certain specific embodiments of the present invention are illustrated by the following representative examples.

## Example 1

In this example, 30 ml of a seed medium (pH 6.0) comprising 0.5 g/dl glucose, 0.4 g/dl yeast extract, 1 g/dl malt extract and 1 mg/dl ferric chloride is put into a 300 ml-Erlenmeyer flask and sterilized at 120°C for 15 minutes. One loopful of *Coriolus versicolor* ATCC 20155 is inoculated into the seed medium and cultured with shaking at 30°C for 5 days. The, 30 ml of the seed culture liquor thus obtained is put into a 2 l-Erlenmeyer flask containing 300 ml of a culture medium comprising the same components as the first seed medium and cultured with shaking at 30°C for 4 days. Thereafter, 1.2 l of the second seed culture liquor is poured into a 30 l-jar fermenter containing 18 l of a fermentation medium comprising the same components as the seed medium. Main fermentation is carried out with aeration-stirring at an aeration rate of 1 l/min., a stirring speed of 250 rpm and 30°C for 2 days. The enzymatic activity of pyranose oxidase

contained in the resulting culture liquor is 161 mU based upon 1 ml of the culture liquor. After the completion of culturing, the culture liquor is subjected to filtration by means of suction, whereby 1 kg (wet weight) of microbial cells is obtained. The cells are suspended in 1.8 l of 50 mM tris-hydrochloride buffer (pH 7.0) (the buffer solutions used hereinafter are the same as this buffer) and then disrupted by homogenizer. The disrupted cells are centrifuged to obtain 1.8 l of supernatant. The supernatant is adjusted to pH 7.0 while adding alkali thereto and passed through a column of one l of HPA—75 pre-equilibrated with the buffer (product of Mitsubishi Kasei Co., Ltd., Japan). After the column is washed with the buffer and successively with the buffer containing 0.1 M ammonium sulfate, elution is carried out with the buffer containing 0.25 M ammonium sulfate. The active fractions of the enzyme are combined and mixed with ammonium sulfate to 80% saturation. The formed precipitate is recovered by centrifugation and dissolved in 30 ml of the buffer. Then, the resulting solution is charged on a column of one l of Sephalose 4B and elution is carried out with the buffer. The active reactions of the enzyme are combined and mixed with ammonium sulfate to 80% saturation. The formed precipitate is recovered by centrifugation and dissolved in 30 ml of the buffer. The resulting enzyme solution is again passed through a column of 500 ml of Sephadex G—100, and elution is carried out with the buffer. The active fractions are freeze-dried whereby 815 mg of an enzyme preparate is obtained (specific activity: 9.6 U/mg protein). The yield in terms of activity based on the supernatant obtained by eluting the disrupted cell suspension is 43%.

Example 2

In this example, *Gloeophyllum sepiarium* Z—41 is inoculated into the medium having the same composition as that of the medium prepared in Example 1 in a 2 l-Erlenmeyer flask and cultured for 6 days. The enzymatic activity of pyranose oxidase contained in the resulting culture liquor is 365 mU/ml.

Example 3

The same culturing as in Example 1 is carried out except that *Daedaleopsis styracina* ATCC 20188 is used as a seed strain. The enzymatic activity of pyranose oxidase contained in the resulting culture liquor is 189 mU/ml.

Example 4

The same culturing as in Example 1 is carried out except that *Pleurotus ostreatus* Z—64 is used as a seed strain. The enzymatic activity of pyranose oxidase contained in the resulting culture liquor is 59 mU/ml.

Example 5

To 0.2 ml of a solution containing glucose in each of concentrations indicated at the measured points of Figure 5, are added 1.5 ml of 0.1 M potassium phosphate buffer (pH 7.5), 0.2 ml of a coloring reagent solution (pH 7.5) containing 10 mmol/l 4AA, 10 mmol/l phenol and 20 U/ml, 0.1 ml of 0.1 mM FAD and 1 ml of a pyranose oxidase solution (6.7 U) to make up 3.0 ml of a reaction solution. The enzymatic reaction solution is incubated at 37°C for 10 minutes. Then, the absorbancy of the solution is measured at 500 nm, the result of which is shown in Figure 5. Proportional relationship between the glucose concentrations and the absorbancies is recognized.

Example 6
Determination of glucose

| | | |
|---|---|---|
| *Reagent 1* | Ascorbate oxidase | 5 U/ml |
| | Triton X—100 (Registered Trade Mark) | 0.15% |
| | Adekatol LO—5 | 0.075% |
| | DHSA | 0.4 mg/ml |
| | 50 mM Borax-100 mM $KH_2PO_4$ (pH 6.5) | |
| *Reagent 2* | 4AA | 0.15 mg/ml |
| | Peroxidase | 7 U/ml |
| | Pyranose oxidase | 3.5 U/ml |
| | Triton X—100 | 0.15% |
| | Adekatol LO—5 | 0.075% |
| | HCl-semicarbazide | 0.4 mg/ml |
| | 50 mM Borax-100 mM $KH_2PO_4$ (pH 6.5) | |

*Procedure*

Test tube
← 1.5 ml of Reagent 1
← 20 µl of Serum sample, Standard solution or Distilled water
↓
Pre-incubation (37°C, 5 min)
← 1.5 ml of Reagent 2
↓
Incubation (37°C, 5 min)
Measurement of absorbancy of the solution at 585 nm (E) (Control: Distilled water)

$$G\ (mg/dl) = \frac{E_S - E_W}{E_{ST} - E_W} \times C\ (mg/dl)$$

G : glucose concentration in sample
$E_S$ : absorbance value of sample
$E_{ST}$ : absorbance value of standard solution
$E_W$ : absorbance value of distilled water
C : concentration of standard solution

*Results*

| | ΔE | G (mg/dl) |
|---|---|---|
| Standard solution (200 mg/dl) | 0.459 | — |
| Sample No. 1 | 0.652 | 284 |
| Sample No. 2 | 1.074 | 468 |
| Sample No. 3 | 0.681 | 297 |
| Sample No. 4 | 0.976 | 425 |
| Sample No. 5 | 0.505 | 220 |
| Sample No. 6 | 0.311 | 136 |
| Sample No. 7 | 0.793 | 346 |
| Sample No. 8 | 0.222 | 97 |
| Sample No. 9 | 0.150 | 65 |
| Sample No. 10 | 0.178 | 78 |

Example 7
Determination for amylase

| | | |
|---|---|---|
| *Reagent 1* | EMAE | 1.2 mg/ml |
| | Maltopentose | 8.05 mg/ml |
| | HCl-semicarbazide | 0.4 mg/ml |
| | Triton X—100 | 0.15% |
| | Catalase | 35.2 U/ml |
| | Pyranose oxidase | 54 U/ml |
| | Maltosephosphorylase | 8 U/ml |
| | 50 mM Borax-100 mM $KH_2PO_4$ | (pH 6.5) |
| *Reagent 2* | 4AA | 0.134 mg/ml |
| | $NaN_3$ | 0.066 mg/ml |
| | Peroxidase | 13.4 U/ml |
| | Triton X—100 | 0.15% |
| | 50 mM Borax-100 mM $KH_2PO_4$ | (pH 6.5) |

*Procedures*

Test tube
↓ ← 1.5 ml of Reagent 1
Pre-incubation (37°C, 5 min)
| ← 50 μl of serum sample, standard serum or distilled water
Incubation (37°C, 5 min)
|
Cell
↓ ← 1.5 ml Reagent 2
Measurement of absorbancy 1 and 55 minutes after the addition of Reagent 2

$$G\ (U/l) = \frac{(E_{S5} - E_{S1}) - (E_{W5} - E_{W1})}{(E_{ST5} - E_{ST1}) - (E_{W5} - E_{W1})} \times C$$

G : amylase activity

*Results*

| | ΔE/min | G (U/l) | | ΔE/min | G (U/l) |
|---|---|---|---|---|---|
| Standard serum | 0.0175 | 470 | Sample No. 6 | 0.005 | 134 |
| Sample No. 1 | 0.013 | 349 | Sample No. 7 | 0.0274 | 736 |
| Sample No. 2 | 0.0076 | 204 | Sample No. 8 | 0.0112 | 301 |
| Sample No. 3 | 0.0084 | 226 | Sample No. 9 | 0.0062 | 167 |
| Sample No. 4 | 0.007 | 188 | Sample No. 10 | 0.0084 | 226 |
| Sample No. 5 | 0.017 | 457 | | | |

**Claims**

1. Pyranose oxidase obtained from a microorganism of the genus *Coriolus, Daedaleopsis, Gloeophyllum* or *Pleurotus* and having the following characteristics:
i) oxidation activity restricted substantially to glucose;
ii) pH stability range: 5.0 to 7.4;
iii) optimum pH about 6.2 (trisbuffer);
iv) optimum temperature: 50°C;
v) isoelectric point: 4.2;
vi) molecular weight: about 220,000;
vii) Km value for glucose: 0.83 mM.

2. A pyranose oxidase according to claim 1, obtained from a microorganism of the species *Coriolus veriscolor, Daedaleopsis styracina, Gloeophyllum sepiarium* or *Pleurotus ostreatus.*

3. A pyranose oxidase according to claim 1, obtained from *Coriolus veriscolor* ATCC 20155, *Daedaleopsis styracina* ATCC 20188, *Gloeophyllum sepiarium* NRRL 12506 or *Pleurotus ostreatus* NRRL 12507.

4. A process for producing a pyranose oxidase according to claim 1, which comprises culturing a microorganism belonging to the genus *Coriolus, Daedaleopsis, Gloeophyllum* or *Pleurotus* capable of producing said pyranose oxidase intracellularly, said culturing taking place in a nutrient medium at a temperature in the range 20°C to 35°C for 1 to 7 days at about neutral pH, rupturing the cultured microorganism cells to release the intracellularly produced pyranose oxidase, and recovering the released oxidase from the ruptured cells.

5. A process according to claim 4, wherein said microorganism is as defined in claim 2 or 3.

6. A method for the determination of glucose in a sample, which comprises oxidising the glucose with pyranose oxidase in the presence of oxygen, and determining the amount of a reaction product produced,

or the amount of oxygen consumed, characterised in that the pyranose oxidase used is an enzyme as defined in any one of claims 1—3.

7. A method according to claim 6, wherein the glucose is produced in said sample by the reaction or decomposition of a glucose-producing precursor.

8. A method according to claim 6 or 7, wherein said enzymatic reaction is carried out in a buffer solution.

9. A method according to any one of claims 6—8, wherein the determination is carried out by determining the amount of hydrogen peroxide produced by the enzymatic reaction.

10. A method according to claim 9, wherein said hydrogen peroxide is determined by reacting said hydrogen peroxide with a chromogen in the presence of peroxidase to form a pigment and then measuring the amount of pigment formed colorimetrically.

11. A test kit or composition for the determination of glucose in a sample which comprises (a) a pyranose oxidase-containing composition for oxidizing the glucose in said sample, and (b) a composition or apparatus for determining the amount of a product of said oxidation reaction, or the amount of oxygen consumed, wherein the pyranose oxidase component is a pyranose oxidase as defined in any one of claims 1—3.

12. A test kit or composition according to claim 11, wherein component (b) is a reagent composition comprising a peroxidase and a chromogen.

13. A test kit or composition according to claim 12, wherein said chromogen is 4AA-phenol, 4AA-DMA, 4AA-DEA, 4AA-EMAE, 4AA-DBA, 4AA-DHSA, MBTH-phenol, MBTH-DEA or MBTH-DBA.

**Patentansprüche**

1. Pyranose-Oxidase, erhalten aus einem Mikroorganismus der Gattung Coliolus, Daedaleopsis, Gloeophyllum oder Pleurotus, mit den folgenden Eigenschaften:
i) Oxidationsaktivität im wesentlichen beschränkt auf Glucose;
ii) pH-Stabilitätsbereich: 5,0 bis 7,4;
iii) pH-Optimum bei etwa 6,2 (Trispuffer);
iv) Temperaturoptimum: 50°C;
v) Isoelektrischer Punkt: 4,2;
vi) Molekulargewicht: etwa 220 000;
vii) $K_m$-Wert für Glucose: 0,83 mM.

2. Pyranose-Oxidase nach Anspruch 1, erhalten aus einem Mikroorganismus der Gattung Coriolus veriscolor, Daedaleopsis styracina, Gloeophyllum sepiarium oder Pleurotus ostreatus.

3. Pyranose-Oxidase nach Anspruch 1, erhalten aus Coriolus veriscolor ATCC 20155, Daedaleopsis styracina ATCC 20188, Gloeophyllum sepiarium NRRL 12506 oder Pleurotus ostreatus NRRL 12507.

4. Verfahren zur Herstellung einer Pyranose-Oxidase nach Anspruch 1, bei dem man einen Mikroorganismus der Gattung Coriolus, Daedaleopsis, Gloeophyllum oder Pleurotus züchtet, der zur intrazellulären Bildung der Pyranose-Oxidase fähig ist, wobei das Züchten in einem Kulturmedium 1 bis 7 Tage bei einer Temperatur im Bereich von 20°C bis 35°C und etwa neutralem pH-Wert stattfindet, die gezüchteten Mikroorganismus-Zellen zur Freisetzung der intrazellulär gebildeten Pyranose-Oxidase aufbricht, und die freigesetzte Oxidase aus den aufgebrochenen Zellen gewinnt.

5. Verfahren nach Anspruch 4, bei dem der Mikroorganismus der in Anspruch 2 oder 3 definierte ist.

6. Verfahren zur Bestimmung von Glucose in einer Probe, bei dem man die Glucose mit Pyranose-Oxidase in Gegenwart von Sauerstoff oxidiert und die Menge des gebildeten Reaktionsproduktes oder die Menge des verbrauchten Sauerstoffs bestimmt, dadurch gekennzeichnet, daß die verwendete Pyranose-Oxidase ein wie in einem der Ansprüche 1 bis 3 definiertes Enzym ist.

7. Verfahren nach Anspruch 6, wobei die Glucose in der Probe durch die Umsetzung oder den Abbau einer Glucose-bildenden Vorstufe hergestellt wird.

8. Verfahren nach Anspruch 6 oder 7, wobei die enzymatische Umsetzung in einer Pufferlösung durchgeführt wird.

9. Verfahren nach einem der Ansprüche 6 bis 8, wobei die Bestimmung durch Bestimmung der Wasserstoffperoxid-Menge erfolgt, die durch die enzymatische Umsetzung gebildet wurde.

10. Verfahren nach Anspruch 9, wobei das Wasserstoffperoxid bestimmt wird durch Umsetzung des Wasserstoffperoxids mit einem Chromogen in Gegenwart von Peroxidase zur Bildung eines Pigments und anschließende colorimetrische Messung der gebildeten Pigmentmenge.

11. Testbesteck oder Zusammenstellung zur Bestimmung von Glucose in einer Probe, umfassend (a) ein Pyranose-Oxidase-enthaltendes Mittel zur Oxidation der Glucose in der Probe und (b) ein Mittel oder eine Vorrichtung zur Bestimmung der Produktmenge der Oxidationsreaktion oder der verbrauchten Sauerstoffmenge, wobei der Pyranose-Oxidase-Bestandteil eine wie in einem der Ansprüche 1 bis 3 definierte Pyranose-Oxidase ist.

12. Testbesteck oder Zusammenstellung nach Anspruch 11, wobei der Bestandteil (b) eine Reagenzzusammenstellung ist, die eine Peroxidase und ein Chromogen enthält.

13. Testbesteck oder Zusammenstellung nach Anspruch 12, wobei das Chromogen 4AA-Phenol, 4AA-DMA, 4AA-DEA, 4AA-EMAE, 4AA-DBA, 4AA-DHSA, MBTH-Phenol, MBTH-DEA oder MBTH-DBA ist.

**Revendications**

1. Pyranose-oxydase obtenue à partir d'un microorganisme du genre *Coriolius, Daedaleopsis, Gloeophyllum* ou *Pleurotus* et possèdant les caractéristiques suivantes:
i) activité d'oxydation pratiquement limitée au glucose;
ii) plage de pH pour la stabilité: 5,0 à 7,4;
iii) pH optimal d'environ 6,2 (tampon Tris);
iv) température optimale: 50°C;
v) point isoélectrique: 4,2;
vi) masse moléculaire: environ 220 000;
vii) valeur Km pour le glucose: 0,83 mM.

2. Pyranose-oxydase selon la revendication 1, obtenue à partir d'un microorganisme de l'espèce *Coriolus veriscolor, Daedaleopsis styracina, Gloeophyllum sepiarium* ou *Pleurotus ostreatus*.

3. Pyranose-oxydase selon la revendication 1, obtenue à partir de *Coriolius veriscolor* ATCC 20155, *Daedaleopsis styracina* ATCC 20188, *Gloeophyllum sepiarium* NRRL 12506 ou *Pleurotus ostreatus* NRRL 12507.

4. Procédé pour la production d'une pyranose-oxydase selon la revendication 1, qui comprend la culture d'un microorganisme appartenant au genre *Coriolus, Daedaleopsis, Gloeophyllum* ou *Pleurotus* capable de produire ladite pyranose-oxydase de manière intracellulaire, ladite culture s'effectuant dans un milieu nutritif à une température comprise dans l'intervalle allant de 20°C à 35°C pendant de 1 à 7 jours, à un pH pratiquement neutre, l'éclatement des cellulose du microorganisme cultivé pour libérer la pyranose-oxydase produite de manière intracellulaire et la récupération de l'oxydase libérée hors des cellules éclatées.

5. Procédé selon la revendication 4, dans lequel ledit microorganisme est tel que défini dans la revendication 2 ou 3.

6. Procédé pour le dosage de glucose dans un échantillon, qui comprend l'oxydation du glucose avec la pyranose-oxydase en présence d'oxygène, et la détermination de la quantité d'un produit de réaction produit ou de la quantité d'oxygène consommée, caractérisé en ce que la pyranose-oxydase utilisée est une enzyme telle que définie dans l'une quelconque des revendications 1 à 3.

7. Procédé selon la revendication 6, dans lequel le glucose est produit dans ledit échantillon par la réaction ou la décomposition d'un précurseur produisant du glucose.

8. Procédé selon la revendication 6 ou 7, dans lequel on effectue ladite réaction enzymatique dans une solution tampon.

9. Procédé selon l'une quelconque des revendications 6 à 8, dans lequel on effectue le dosage par détermination de la quantité de peroxyde d'hydrogène produit par la réaction enzymatique.

10. Procédé selon la revendication 9, dans lequel on dose ledit peroxyde d'hydrogène par réaction dudit peroxyde d'hydrogène avec un colorant chromogène en présence de peroxydase pour former un pigment, puis mesure de la quantité de pigment formé par colorimétrie.

11. Composition ou trousse d'analyse pour le dosage du glucose dans un échantillon qui comprend (a) une composition contenant la pyranose-oxydase pour oxyder le glucose dans ledit échantillon et (b) une composition ou un appareil pour déterminer la quantité d'un produit de ladite réaction d'oxydation, ou la quantité d'oxygène consommée, où le constituant pyranose-oxydase est une pyranose-oxydase telle que définie dans l'une quelconque des revendications 1 à 3.

12. Composition ou trousse d'analyse selon la revendication 11, dans lequel le constituant (b) est une composition de réactifs comprenant une peroxydase et un colorant chromogène.

13. Composition ou trousse d'analyse selon la revendication 12, dans lequel ledit colorant chromogène est: 4AA-phénol, 4AA-DMA, 4AA-DEA, 4AA-EMAE, 4AA-DBA, 4AA-DHSA, MBTH-phénol, MBTH-DEA ou MBTH-DBA.

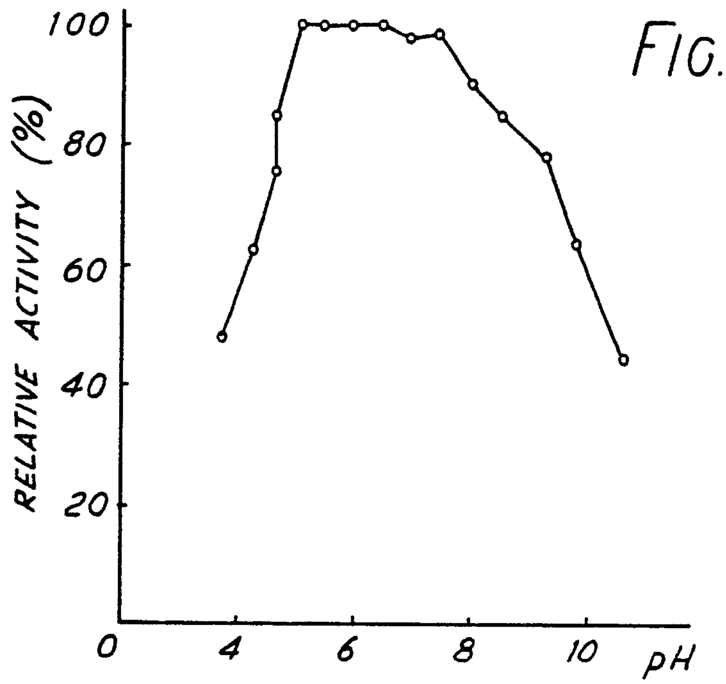
100
80
60
40
20
RELATIVE ACTIVITY (%)
0
4
6
8
10
pH

FIG.1

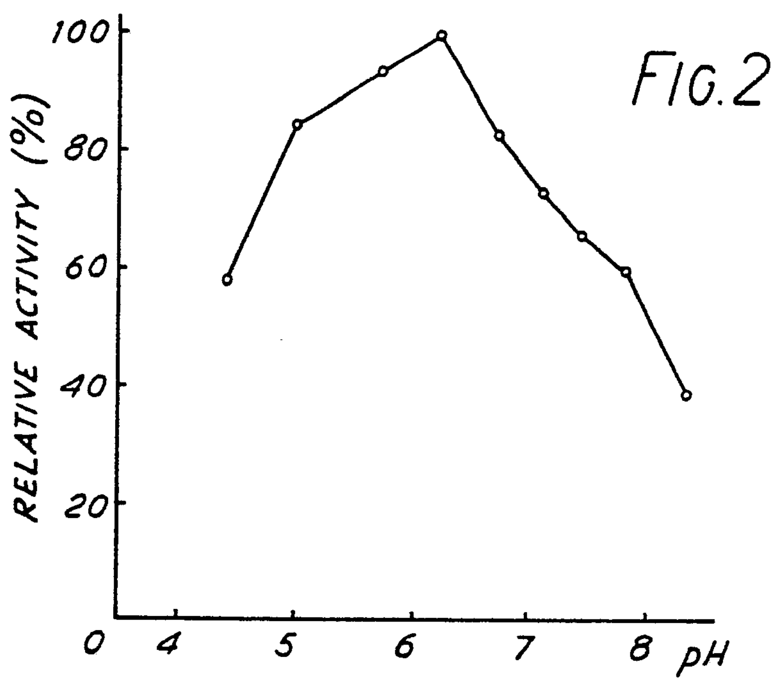
100
80
60
40
20
RELATIVE ACTIVITY (%)
0
4
5
6
7
8
pH

FIG.2

FIG. 3

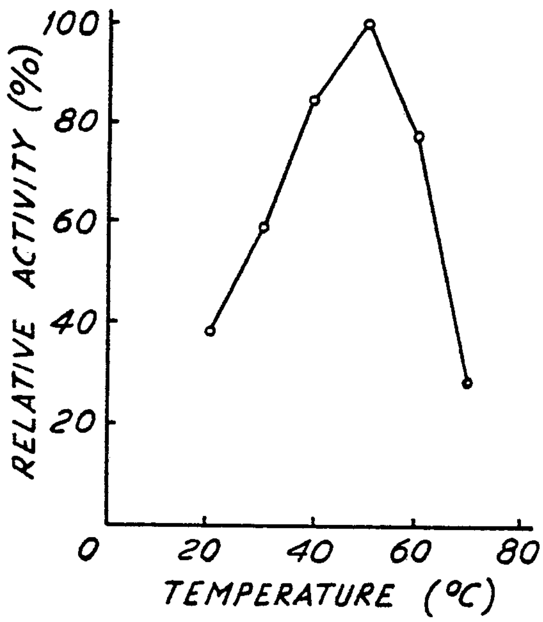


FIG. 4

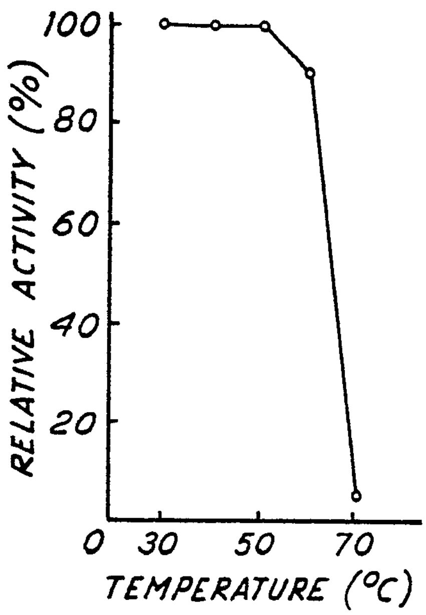


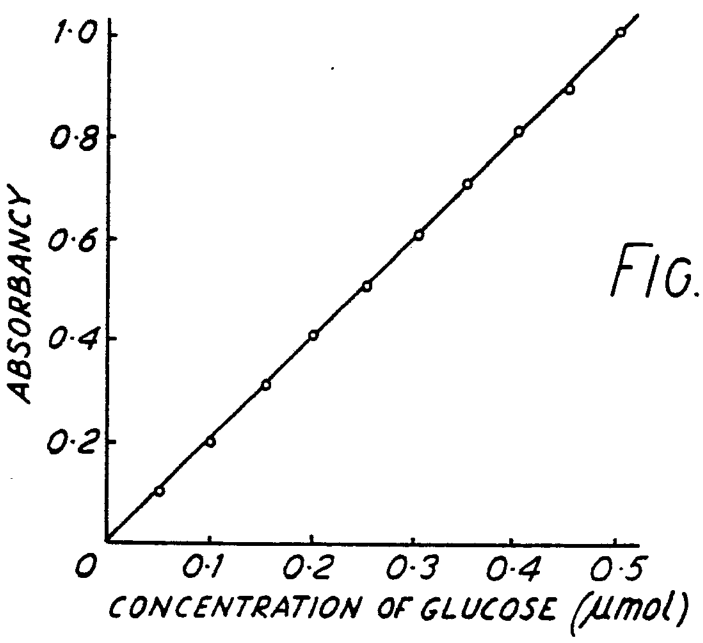


FIG. 5